(19) Europäisches Patentamt
European Patent Office
Office européen des brevets

(11) EP 1 785 128 B1

(12) FASCICULE DE BREVET EUROPEEN

(45) Date de publication et mention de la délivrance du brevet:
**24.12.2008 Bulletin 2008/52**

(51) Int Cl.:
***A61K 8/04*** *(2006.01)* ***A61Q 1/10*** *(2006.01)*

(21) Numéro de dépôt: **06123083.5**

(22) Date de dépôt: **27.10.2006**

(54) **Composition de revêtement des cils sous forme de mousse**

Schaumförmige Beschichtungszusammensetzung für Augenwimpern

Composition in the foam form for coating the eyelashes

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **09.11.2005 FR 0553406**

(43) Date de publication de la demande:
**16.05.2007 Bulletin 2007/20**

(73) Titulaire: **L'Oréal**
**75008 Paris (FR)**

(72) Inventeurs:
- **Jager Lezer, Nathalie**
**91370, Verrieres-le-Buisson (FR)**
- **Arditty, Stéphane**
**91160, Ballainvilliers (FR)**
- **Thiebaut, Laure**
**92110, Clichy (FR)**

(74) Mandataire: **Duvert, Sandra**
**L'OREAL**
**25-29 Quai Aulagnier**
**92665 Asnières-sur-Seine (FR)**

(56) Documents cités:
**EP-A- 1 407 755** **WO-A-20/04060292**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 785 128 B1

## Description

La présente invention se rapporte à un procédé de revêtement des cils consistant à appliquer sur les cils une composition sous forme de mousse.

La composition peut se présenter sous la forme d'un mascara, d'un produit pour les sourcils. Plus spécialement, l'invention porte sur un mascara.

Par mascara, on entend une composition destinée à être appliquée sur les cils : il peut s'agir d'une composition de maquillage des cils, une base de maquillage des cils (aussi appelée base coat), une composition à appliquer sur un mascara, dite encore top-coat, ou bien encore une composition de traitement cosmétique des cils. Le mascara est plus particulièrement destiné aux cils d'êtres humains, mais également aux faux-cils.

D'une manière générale, les compositions de maquillage des cils ou mascaras sont constituées d'au moins une cire ou d'un mélange de cires dispersé dans une phase liquide aqueuse ou solvant organique. Ils présentent en général une texture pâteuse (voir par example le document EP 1 407 755 A) et sont conditionnés dans un récipient comprenant un réservoir muni d'un essoreur et d'un applicateur, notamment sous forme d'une brosse ou d'un peigne, et qui s'appliquent par prélèvement du produit dans le réservoir à l'aide de l'applicateur, passage de l'applicateur au travers de l'essoreur afin d'évacuer le surplus de produit, puis mise en contact de l'applicateur imprégné de mascara sur les cils.

Il est également connu par exemple des documents US 2 007 245 ou FR 2 833 163 des mascaras sous forme solide dits aussi « mascaras pains » qui sont des compositions comportant une forte proportion de cires, de pigments et de tensioactifs, délitables à l'eau c'est à dire qu'ils nécessitent, préalablement à leur application sur les cils, la mise en contact avec une phase aqueuse de manière à solubiliser partiellement le pain de mascara. En particulier l'application se fait via une brosse imprégnée d'eau qui est mise en contact avec le mascara puis le mélange prélevé et ensuite appliqué sur les cils avec la brosse de manière à déposer de la matière sur les cils.

La présente invention a pour but de proposer une autre voie de formulation pour une composition de revêtement des cils qui présente une texture de mousse.

Par composition sous forme de mousse, on entend une composition comprenant une phase gazeuse (par exemple de l'air) sous forme de bulles.

La composition sous forme de mousse présente une texture légère, facile à prélever et à étaler sur les cils. Elle permet notamment l'obtention d'un effet maquillage particulier dits « cils étoilés » par la formation de crêtes dans la frange de cils, dues à l'agglomération de groupes de cils entre eux à leur extrémité supérieure.

Le procédé de revêtement des cils selon l'invention consiste à appliquer sur les cils la composition sous forme de mousse, il se distingue des procédés de l'art antérieur (voir par example le document WO 2004/060292 A2) en ce que la mousse ne se forme pas in situ sur les cils, c'est-à-dire que la mousse ne se crée pas après application de ladite composition. En particulier, il ne s'agit pas d'une composition à expansion retardée qui est un système dans lequel un agent dit volatil est libéré ou formé dans la composition après que celle-ci ait été appliquée sur les cils. Spécifiquement, les compositions à expansion retardée sont crées après exposition d'un gel à la pression atmosphérique, et/ou à un cisaillement et/ou à une température supérieure à la température ambiante.

De façon plus précise, l'invention a pour objet un procédé de revêtement des cils comprenant l'application sur lesdits cils d'au moins une couche d'au moins une composition sous forme de mousse, ladite composition présentant une densité inférieure ou égale à 0,95 et un module de rigidité plateau Gp inférieur à 50 000 Pa.

L'invention a aussi pour objet un procédé de revêtement des cils comprenant l'application sur lesdits cils d'au moins une couche d'au moins une composition sous forme de mousse, ladite composition présentant une densité inférieure ou égale à 0,95 et une teneur en matière sèche inférieure ou égale à 60 % en poids par rapport au poids total de la composition.

Selon un mode de réalisation, la composition comprend une phase grasse liquide et un agent structurant de phase grasse. C'est pourquoi l'invention a également pour objet un procédé de revêtement des cils comprenant l'application sur lesdits cils d'au moins une couche d'au moins une composition sous forme de mousse, ladite composition présentant une densité inférieure ou égale à 0,95 et comprenant une phase grasse liquide et au moins un agent structurant de phase grasse.

Selon un autre aspect, la composition comprend une phase aqueuse et au moins un gélifiant hydrophile. C'est pourquoi un autre objet de l'invention est un procédé de revêtement des cils comprenant l'application sur lesdits cils d'au moins une couche d'au moins une composition sous forme de mousse, ladite composition présentant une densité inférieure ou égale à 0,95 et comprenant une phase aqueuse et au moins un gélifiant hydrophile.

L'invention a aussi pour objet un kit de maquillage et/ou de soin non thérapeutique des cils comprenant :

- une composition sous forme de mousse présentant une densité inférieure ou égale à 0,95 et
- un applicateur comportant au moins un élément d'application configuré pour appliquer la composition sur les cils.

## Densité

La densité est mesurée selon le protocole suivant : un récipient dont le volume Vo ($cm^3$) est connu avec une précision de ± 0.005 $cm^3$ (Vo étant de l'ordre de 10 $cm^3$), est pesé au moyen d'une balance de précision à ± 0.00005g. Sa masse est notée Mo (g). Ce récipient est rempli délicatement avec la mousse jusqu'au débordement du récipient. La surface du récipient est alors arasée avec une lame droite afin d'obtenir une surface de mousse parfaitement plane. On mesure alors la masse M (g) du récipient rempli de mousse.

La densité correspond au rapport entre la masse volumique de la composition calculée comme suit :

$$\rho_v(g/cm^3) = \frac{M - Mo}{Vo}$$

sur la masse volumique de l'eau (1 g/$cm^3$).

La composition mise en oeuvre dans le procédé selon l'invention présente une densité inférieure ou égale à 0,95, de préférence inférieure ou égale à 0,9, mieux, inférieure ou égale à 0,8. De préférence, la densité de la composition est supérieure ou égale à 0,1, et mieux supérieure ou égale à 0,2 .

## Extrait Sec

Les compositions selon l'invention présentent avantageusement une teneur en matière sèche (ou extrait sec) inférieure ou égale à 60 % en poids par rapport au poids total de la composition, de préférence inférieure ou égale à 55% en poids, mieux inférieure ou égale à 50% en poids.

L'extrait sec est de préférence supérieure ou égale à 10% en poids. Il va en particulier de 35 à 50% en poids par rapport au poids total de la composition.

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec » des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

On étale environ 1g de la composition sur une coupelle métallique. Celle-ci, après introduction dans le dessiccateur, est soumise à une consigne de température de 120 °C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante :

Extrait Sec = 100 x (masse sèche / masse humide).

## Caractérisations rhéologiques

Les compositions conformes à l'invention ont avantageusement un comportement viscoélastique.

De façon générale, un matériau est dit viscoélastique quand, sous l'effet du cisaillement, il possède à la fois les caractéristiques d'un matériau élastique, c'est à dire capable de stocker de l'énergie et les caractéristiques d'un matériau visqueux, c'est à dire capable de dissiper de l'énergie.

Le comportement viscoélastique des compositions conformes à l'invention peut être plus particulièrement caractérisé par son module de rigidité G. Ce paramètre est notamment défini dans l'ouvrage "Initiation à la rhéologie", G. Couarraze et J.L. Grossiord, 2ème édition, 1991, Edition Lavoisier-Tec 1 Doc.

Les mesures sont effectuées sur un rhéomètre à contrainte imposée, RS 600 de la société ThermoRhéo, équipé d'un bain thermostaté et d'un mobile en acier inoxydable à géométrie plan/plan, le plan ayant un diamètre de 20 mm et un entrefer (distance entre le plan inférieur - appelé plan stator - sur lequel est déposée la composition et le

plan supérieur - appelé plan rotor) de 1 mm. Les 2 plans sont striés pour limiter les phénomènes de glissement aux parois des plans. On prend soin de charger la mousse en approchant les plans à la vitesse minimale de l'appareil (0.6mm/min) afin de ne pas déstructurer la mousse lors de la charge dans le rhéomètre. Les mesures sont effectuées à 25°C ± 0,5°C.

Les mesures dynamiques sont réalisées en appliquant une variation harmonique de la contrainte. Dans ces expériences, les amplitudes de la contrainte de cisaillement (notée $\tau$) et de la déformation de cisaillement (notée y) sont faibles de manière à rester dans les limites du domaine viscoélastique linéaire de la composition (conditions permettant d'évaluer les caractéristiques rhéologiques de la composition au repos).

Le domaine linéaire viscoélastique est généralement défini par le fait que la réponse du matériau (i.e. la déformation) est à tout moment directement proportionnelle à la valeur de la force appliquée (i.e. la contrainte). Dans ce domaine, les contraintes appliquées sont faibles et le matériau subit des déformations sans modifier sa structure microscopique. Dans ces conditions, le matériau est étudié « au repos » et de façon non destructive.

La composition est soumise à un cisaillement harmonique selon une contrainte $\tau(t)$ variant de façon sinusoïdale selon une pulsation $\omega$ ($\omega = 2\Pi\nu$), $\nu$ étant la fréquence du cisaillement appliqué. La composition ainsi cisaillée subie une contrainte $\tau(t)$ et répond selon une déformation $\gamma(t)$ correspondant à des micro déformations pour lesquelles le module de rigidité varie peu en fonction de la contrainte imposée.

La contrainte $\tau(t)$ et la déformation $\gamma(t)$ sont définies respectivement par les relations suivantes :

$$\tau(t) = \tau_0 \cos(\omega \cdot t) \qquad \gamma(t) = \gamma_0 \cos(\omega \cdot t - \delta)$$

$\tau_0$ étant l'amplitude maximale de la contrainte et $\gamma_0$ étant l'amplitude maximale de la déformation. L'élasticité $\delta$ est l'angle de déphasage entre la contrainte et la déformation.

Les mesures sont effectuées à une fréquence de 1 Hz ($\nu$= 1 Hz).

On applique des contraintes croissantes à l'échantillon en partant d'une contrainte initiale égale à 3 Pa pour arriver à une contrainte finale de 4000 Pa, les contraintes n'étant appliquées qu'une seule fois

On mesure ainsi l'évolution du module de rigidité G (correspondant au rapport de $\tau_o$ sur $\gamma_o$) et de l'élasticité $\delta$ (correspondant à l'angle de déphasage de la contrainte appliquée par rapport à la déformation mesurée) en fonction de la contrainte $\tau(t)$ appliquée.

On mesure en particulier la déformation de la composition pour la zone de contrainte dans laquelle la variation du module de rigidité G et de l'élasticité $\delta$ est inférieure à 7 % (zone des microdéformations) et on détermine ainsi le paramètres dit « plateau » Gp.

Le comportement viscoélastique des compositions selon l'invention est notamment caractérisé par un module de rigidité plateau Gp inférieur à 50 000 Pa, voire inférieur ou égal à 25 000 Pa, notamment inférieur ou égal à 15 000 Pa et mieux inférieur ou égal à 10 000 Pa.

En particulier, les compositions selon l'invention possèdent un module de rigidité plateau Gp supérieur ou égal à 100 Pa, voir supérieur ou égal à 300 Pa.

Taille des bulles

La composition selon l'invention étant sous forme de mousse, elle comprend des bulles de gaz, avantageusement des bulles d'air.

En particulier, les bulles d'air de la composition présentent un diamètre moyen en nombre inférieur ou égal à 1 mm, allant par exemple de 0,1 $\mu$m à 1 mm, de préférence inférieur ou égal à 0,8 mm.

Le diamètre moyen en nombre est déterminé de la façon suivante : la mousse est conditionnée en pot dès la fin de la préparation, lorsqu'elle est encore fluide. L'observation de la taille des bulles est effectuée en prenant une photo numérique de la surface du pot 24h après fabrication ou après ouverture du conditionnement après avoir arasé la surface , puis, à l'aide du logiciel de traitement d'image « Saisam », en comptant sur une surface d'environ 1cm$^2$ le nombre de bulles et en déterminant leur diamètre moyen en nombre.

De préférence, la composition mise en oeuvre dans le procédé selon l'invention est une composition non rincée.

**Phase grasse**

La composition selon l'invention peut comprendre une phase grasse de la composition et en particulier une phase grasse liquide.

Par phase grasse liquide, au sens de la demande, on entend une phase grasse liquide à température ambiante (25°C) et pression atmosphérique (760 mm de Hg), composée d'un ou plusieurs corps gras non aqueux liquides à

température ambiante, appelés aussi huiles, compatibles entre eux.

L'huile peut être choisie parmi les huiles volatiles et/ou les huile non volatiles, et leurs mélanges.

La phase grasse liquide peut représenter de 0,1 % à 30 % en poids, de préférence de 1 % à 20 % en poids par rapport au poids total de la composition.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'isohexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I)

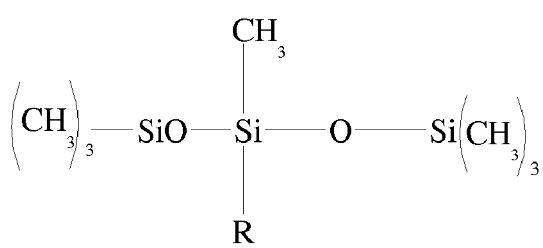

où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.

La composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

Selon un mode de réalisation, la phase grasse contient une huile ester. Cette huile ester peut être choisie parmi les esters des acides monocarboxyliques avec les monoalcools et polyalcools.

Avantageusement, ledit ester répond à la formule (I) suivante :

$$R_1\text{-CO-O-}R_2 \qquad (I)$$

où $R_1$ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

$R_2$ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que $R_1$ et ou $R_2$ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de $R_1 + R_2$ est ≥ 9.

$R_1$ peut représenter le reste d'un acide gras, de préférence supérieur, linéaire ou, de préférence ramifié comprenant de 1 à 40 et mieux de 7 à 19 atomes de carbone et $R_2$ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de $R_1 + R_2 \geq 9$.

Des exemples des groupes $R_1$ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, éléostéarique, arachidonique, érucique, et de leurs mélanges.

Des exemples d'esters sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule (I) ci-dessus, dans laquelle $R_1$ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et $R_2$ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, $R_1$ est un groupe alkyle ramifié non substitué de 4 à 14 atomes de carbone, de préférence de 8 à 10 atomes de carbone et $R_2$ est un groupe alkyle ramifié non substitué de 5 à 15 atomes de carbone, de préférence de 9 à 11 atomes de carbone. De préférence dans la formule (I), $R_1$-CO- et $R_2$ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :

- l'isononanoate d'isononyle,
- l'octanoate de cétostéaryle,
- le myristate d'isopropyle,
- le palmitate d'éthyl-2-hexyle,
- le stéarate d'octyl 2-dodécyle,
- l'érucate d'octyl 2-dodécyle,
- l'isostéarate d'isostéaryle.

Agent structurant

La composition selon l'invention peut comprendre un agent structurant de phase grasse liquide choisi parmi les corps gras pâteux, les polymères semi-cristallin, les gélifiants lipophiles et leur mélanges.

L'agent structurant peut représenter de 0,1 à 60 % en poids par rapport au poids total de la composition, de préférence de 0,5 à 50 % et de façon encore plus préférée de 1 à 40 % en poids.

La quantité en structurant huileux peut être ajustée par l'homme du métier en fonction du propriétés de structuration desdits agents.

*Corps gras pâteux*

Par corps gras pâteux, on entend un composé gras lipophile comportant à la température de 23°C une fraction liquide et une fraction solide.

Ledit composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1 mm/s puis à une vitesse de mesure de 0,1 mm/s, la profondeur de pénétration étant de 0,3 mm. La valeur relevée de la dureté est celle du pic maximum.

La fraction liquide du composé pâteux mesurée à 23°C représente de préférence 9 à 97% en poids du composé. Cette fraction liquide à 23°C représente de préférence entre 15 et 85%, de préférence encore entre 40 et 85% en poids. La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Les corps pâteux sont généralement des composés hydrocarbonés comme les lanolines et leurs dérivés ou encore des PDMS.

*Polymères semi-cristallins*

On entend par polymère des composés comportant au moins deux motifs, de préférence au moins 3 motifs et plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30°C (notamment allant de 30°C à 80°C), de préférence allant de 30°C à 60°C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquels s'applique l'invention présentent une masse moléculaire moyenne en nombre supérieure ou égale à 1 000. De façon avantageuse, le ou les polymères semi-cristallins de la composition de l'invention ont une masse moléculaire moyenne en nombre $\bar{M}n$ allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

De tels polymères sont décrits par exemple dans le document EP 1396259.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C).

*Gélifiants lipophiles*

Les gélifiants utilisables dans les compositions selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée éventuellement traitée hydrophobe en surface dont la taille des particules est inférieure à 1 μm. Il est en effet possible de modifier chimiquement la surface de la silice, par réaction

chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6éme édition, 1995). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

- Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre ($\alpha$) au moins un acide choisi parmi les acides dicarboxyliques comprenant au moins 32 atomes de carbone tels que les acides gras dimères et ($\beta$) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les polyamides siliconés du type polyorganosiloxane tels que ceux décrits dans les documents US-A-5 874 069, US-A-5,919,441, US-A-6,051,216 et US-A-5,981,680 comme par exemple ceux commercialisés sous la référence Dow Corning 2-8179 et Dow Corning 2-8178 Gellant Gellant par la société DOW CORNING ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type "dibloc", "tribloc" ou "radial" du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

<u>**Phase aqueuse**</u>

La composition selon l'invention peut comprendre un milieu aqueux, constituant une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.

La phase aqueuse (eau et éventuellement le solvant miscible à l'eau) peut être présente, en une teneur allant de 1 % à 95 % en poids, par rapport au poids total de la composition, de préférence allant de 3 % à 80 % en poids, et

préférentiellement allant de 5 % à 60 % en poids.

*Système émulsionnant*

Les compositions selon l'invention peuvent contenir des agents tensioactifs émulsionnants présents notamment en une proportion allant de 0,1 à 20 %, et mieux de 0,3 % à 15 % en poids par rapport au poids total de la composition.

Selon l'invention, on utilise généralement un émulsionnant choisi de manière appropriée pour l'obtention d'une émulsion huile-dans-eau. En particulier, on peut utiliser un émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents tensioactifs peuvent être choisis parmi des agents tensioactifs non ioniques, anioniques, cationiques, amphotériques ou encore des émulsionnants tensioactifs. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

Les tensioactifs utilisés préférentiellement dans la composition selon l'invention sont choisis parmi :

a) les agents tensioactifs non ioniques de HLB supérieur ou égal à 8 à 25 °C, utilisés seuls ou en mélange; on peut citer notamment :

les éthers oxyéthylénés et/ou oxypropylénés (pouvant comporter de 1 à 150 groupes oxyéthylénés et/ou oxypropylénés) de glycérol ;

les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 20 groupes oxyéthylénés (nom CTFA "Steareth-20 ") tel que le BRIJ 78 commercialisé par la société UNIQUEMA, l'éther oxyéthyléné de l'alcool cétéarylique à 30 groupes oxyéthylénés (nom CTFA "Ceteareth-30 ") et l'éther oxyéthyléné du mélange d'alcools gras en C12-C15 comportant 7 groupes oxyéthylénés (nom CTFA "C12-15 Pareth-7" commercialisé sous la dénomination NEODOL 25-7® par SHELL CHEMICALS,

les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,

les esters d'acide gras (notamment d'acide en C8-C24, et de préférence en C16-C22) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,

les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60® par la société UNIQUEMA,

la diméthicone copolyol, telle que celle vendue sous la dénomination Q2-5220® par la société DOW CORNING,

la diméthicone copolyol benzoate (FINSOLV SLB 101® et 201® de la société FINTEX),

les copolymères d'oxyde propylène et d'oxyde d'éthylène, également appelés polycondensats OE/OP,

et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à

10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.

b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus tels que :

les esters et éthers d'oses tels que les stéarate de sucrose, cocoate de sucrose, stéarate de sorbitan et leurs mélanges comme l'Arlatone 2121® commercialisé par la société ICI ou le SPAN 65V de la société UNIQUEMA ;
les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
les éthers oxyéthylénés et/ou oxypropylénés tels que l'éther oxyéthyléné de l'alcool stéarylique à 2 groupes oxyéthylénés (nom CTFA "Steareth-2 ") tel que le BRIJ 72 commercialisé par la société UNIQUEMA ;
le mélange de cyclométhicone/diméthicone copolyol vendu sous la dénomination Q2-3225C® par la société DOW CORNING,

c) Les tensioactifs anioniques tels que :

les sels d'acides gras en C16-C30 notamment ceux dérivant des amines, comme le stéarate de triéthanolamine ;
les sels d'acides gras polyoxyéthylénés notamment ceux dérivant des amines ou les sels alcalins, et leurs mélanges ;
les esters phosphoriques et leurs sels tels que le "DEA oleth-10 phosphate" (Crodafos N 10N de la société CRODA) ou le phosphate de monocétyle monopotassique (Amphisol K de Givaudan ou ARLATONE MAP 160K de la société UNIQUEMA) ;
les sulfosuccinates tels que le "Disodium PEG-5 citrate lauryl sulfosuccinate" et le "Disodium ricinoleamido MEA sulfosuccinate" ;
les alkyléthersulfates tels que le lauryl éther sulfate de sodium ;
les iséthionates ;
les acylglutamates tels que le "Disodium hydrogenated tallow glutamate" (AMISOFT HS-21 R® commercialisé par la société AJINOMOTO) et leurs mélanges.

A titre représentatif des tensioactifs cationiques, on peut notamment citer :

- les alkyl-imidazolidinium tels que l'étho-sulfate d'isostéaryl-éthylimidonium,
- les sels d'ammonium tels que le chlorure de N,N,N-triméthyl-1-docosanaminium (chlorure de Behentrimonium).

Les compositions selon l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

*Gélifiant hydrophile*

La composition selon l'invention peut comprendre un gélifiant hydrophile. Les gélifiants hydrophiles utilisables dans les compositions selon l'invention peuvent être choisi parmi :

les homo- ou copolymères d'acides acrylique ou méthacrylique ou leurs sels et leurs esters et en particulier les produits vendus sous les dénominations VERSICOL F® ou VERSICOL K® par la société ALLIED COLLOID, UTRAHOLD 8® par la société CIBA-GEIGY, les acides polyacryliques de type SYNTHALEN K,
les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,

les copolymères acide polyacryliques/acrylates d'alkyle de type PEMULEN,
l'AMPS (Acide polyacrylamidométhyl propane sulfonique neutralisé partiellement à l'ammoniaque et hautement réticulé) commercialisé par la société CLARIANT,
les copolymères AMPS/acrylamide de type SEPIGEL® ou SIMULGEL® commercialisés par la société SEPPIC, et les copolymères AMPS/méthacrylates d'alkyle polyoxyéthylénés (réticulés ou non),
les protéines comme les protéines d'origine végétale telles que les protéines de blé, de soja ; les protéines d'origine animale tels que les kératines, par exemples les hydrolysats de kératine et les kératines sulfoniques ;
les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
les polymères ou copolymères acryliques, tels que les polyacrylates ou les polyméthacrylates ;
les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
les polymères d'origine naturelle, éventuellement modifiés, tels que :

les gommes arabiques, la gomme de guar, les dérivés du xanthane, la gomme de karaya, la gomme de caroube ;
les alginates et les carraghénanes (les alginates étant utilisées de préférence en présence de sels comme par exemple le chlorure de calcium) ;
les glycoaminoglycanes, l'acide hyaluronique et ses dérivés ;
la résine shellac, la gomme de sandaraque, les dammars, les élémis, les copals ;
l'acide désoxyribonucléïque ;
les muccopolysaccharides tels les chondroïtines sulfate,
et leurs mélanges.

Certains de ces gélifiants hydrosolubles peuvent également jouer le rôle de polymères filmogènes.
Le polymère gélifiant hydrosoluble peut être présent dans la composition selon l'invention en une teneur en matières sèches allant de 0,01% à 30%, mieux 0,05% à 20%, voire 0,1% à 10% en poids par rapport au poids total de la composition.
Les compositions revendiquées peuvent également contenir des ingrédients couramment utilisés dans le domaine du maquillage des cils.

**Cire(s)**

La composition selon l'invention peut avantageusement comprendre au moins une cire.
La cire considérée dans le cadre de la présente invention est d'une manière générale un composé lipophile, solide à température ambiante (25 ˚C), à changement d'état solide/liquide réversible, ayant un point de fusion supérieur ou égal à 30 ˚C pouvant aller jusqu'à 200 ˚C et notamment jusqu'à 120 ˚C.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur ou égal à 45 ˚C, et en particulier supérieur ou égal à 55˚C.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire disposé dans un creuset est soumis à une première montée en température allant de -20 ˚C à 100 ˚C, à la vitesse de chauffe de 10 ˚C/minute, puis est refroidi de 100 ˚C à -20 ˚C à une vitesse de refroidissement de 10 ˚C/minute et enfin soumis à une deuxième montée en température allant de -20 ˚C à 100 ˚C à une vitesse de chauffe de 5 ˚C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.
Les cires susceptibles d'être utilisées dans les compositions selon l'invention sont choisies parmi les cires, solides, à température ambiante d'origine animale, végétale, minérale ou de synthèse et leurs mélanges.
Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

A titre illustratif des cires convenant à l'invention, on peut notamment citer les cires hydrocarbonées comme la cire d'abeille, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en $C_8$-$C_{32}$. Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50®, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A- 2792190.

Selon un mode de réalisation particulier, les compositions selon l'invention peuvent comprendre au moins une cire dite cire collante c'est-à-dire possédant un collant supérieur ou égal à 0,1 N.s et une dureté inférieure ou égale à 3,5 MPa.

La cire collante utilisée peut posséder notamment un collant allant de 0,1 N.s à 10 N.s, en, particulier allant de 0,1 N.s à 5 N.s, de préférence allant de 0,2 à 5 N.s et mieux allant de 0,3 à 2 N.s.

Le collant de la cire est déterminé par la mesure de l'évolution de la force (force de compression) en fonction du temps, à 20 °C selon le protocole indiqué précédemment pour la dureté.

Pendant le temps de relaxation de 1 s, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force. La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange.

Une telle cire est notamment vendue sous les dénominations « Kester Wax K 82 P® », « Hydroxypolyester K 82 P® » et « Kester Wax K 80 P® » par la société KOSTER KEUNEN.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ».

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules de cires, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New

York, 1957.

La taille est exprimée par le diamètre "effectif" moyen en volume D[4,3], défini de la manière suivante :

$$D[4,3] = \frac{\sum_i V_i \cdot d_i}{\sum_i V_i}$$

où $V_i$ représente le volume des particules de diamètre effectif $d_i$. Ce paramètre est notamment décrit dans la documentation technique du granulomètre.

Le diamètre « effectif » est obtenu en prenant un indice de réfraction de 1,33 pour l'eau et un indice de réfraction moyen de 1,42 pour les particules.

Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

La composition selon l'invention peut comprendre une teneur en cires allant de 0,1 à 50 % en poids par rapport au poids total de la composition, en particulier elle peut en contenir de 0,5 à 35 % en poids.

Polymère filmogène

La composition selon l'invention peut comprendre selon un mode de réalisation particulier au moins un polymère filmogène.

Le polymère filmogène peut être présent dans la composition selon l'invention en une teneur en matières sèches (ou matières actives) allant de 0,1 % à 30 % en poids par rapport au poids total de la composition, de préférence de 0,5 % à 20 % en poids, et mieux de 1 % à 15 % en poids.

Dans la présente invention, on entend par « polymère filmogène », un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha$ , $\beta$ -éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$ .

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanespolyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, $K^+$, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est donc solubilisé dans la phase aqueuse de la composition.

Selon une autre variante de réalisation de la composition selon l'invention, le polymère filmogène peut être un

polymère solubilisé dans une phase grasse liquide comprenant des huiles ou solvants organiques tels que ceux décrits précédemment (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisés :

par la société Wacker sous la référence Resin MK tels que la Belsil PMS MK :
par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la

réaction d'un résine de silicone, telle que celles décrite plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER; les sulfopolyesters vendus sous le nom de marque Eastman AQ® par la société Eastman Chemical Products, les dispersions vinyliques comme le Mexomère PAM® de la société CHIMEX et leurs mélanges.

Comme exemples de dispersions non aqueuses de polymère filmogène, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Matière colorante

La composition selon l'invention peut également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Ces matières colorantes peuvent être présentes en une teneur allant de 0,01 à 30 % en poids par rapport au poids total de la composition.

Charges

La composition selon l'invention peut en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydrocarbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL. Les charges peuvent représenter de 0,1 à 25 %, en particulier de 1 à 20 % en poids par rapport au poids total de la composition.

La composition de l'invention peut comprendre, en outre, tout additif usuellement utilisé en cosmétique tels que les antioxydants, les conservateurs, les fibres, les parfums, les neutralisants, les gélifiants, les épaississants, les vitamines, les agents de coalescence, les plastifiants, et leurs mélanges.

Fibres

La composition selon l'invention peut en outre comprendre des fibres qui permettent une amélioration de l'effet allongeant.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Actifs cosmétiques

Comme actifs cosmétiques pouvant être utilisés dans les compositions selon l'invention, on peut citer notamment des antioxydants, les conservateurs, les parfums, les neutralisants, émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

## Procédé de préparation

Les compositions mises en oeuvre dans l'invention peuvent être préparées par des procédés de mélange, d'agitation ou de dispersion de gaz comprimés tels que l'air, les composés à base de chlorofluorocarbone, l'azote, le dioxyde de carbone, l'oxygène, l'hélium, des gaz liquéfiés tels que les gaz de pétrole liquéfiés, un procédé de mélange et d'agitation en présence d'un agent moussant tel qu'un tensioactif.
En particulier, la composition est préparée par mélange, généralement à chaud, des ingrédients sous agitation puis foisonnement sous l'action d'un gaz, la gaz pouvant être introduit lors de l'étape de refroidissement de la composition, ou après préparation de la composition, par exemple à l'aide d'un foisonneur de type Mondomix, d'un batteur de type Kenwood, d'un échangeur à surface raclée ou d'un mélangeur dynamique (de type IMT par exemple). Le gaz est de préférence l'air ou l'azote.
La composition avant foisonnement peut se présenter sous forme de suspension, de dispersion, de solution, de gel, d'émulsion, notamment émulsion huile-dans-eau (H/E), cire-dans-eau ou eau-dans-huile (E/H), ou multiple (E/H/E ou polyol/H/E ou H/E/H), elle peut être anhydre.

## Kit

La composition selon l'invention peut être conditionnée dans un récipient délimitant au moins un compartiment qui comprend ladite composition, ledit récipient étant fermé par un élément de fermeture. Le récipient peut être équipé d'un organe pour la distribution dudit produit. En particulier, le récipient peut être équipé d'une pompe. En variante, il peut être équipé d'une valve, le produit étant alors conditionné sous pression à l'intérieur du récipient. Dans les cas où le récipient est équipé d'un organe de distribution, la présentation du produit sous forme de mousse peut être obtenue au moment de sa distribution.
Le récipient peut être au moins pour partie réalisé en matériau thermoplastique. A titre d'exemples de matériaux thermoplastiques, on peut citer le polypropylène ou le polyéthylène. Alternativement, le récipient est réalisé en matériau non thermoplastique, notamment en verre ou en métal (ou alliage).
Le récipient est de préférence associé à un applicateur comportant au moins un élément d'application configuré pour appliquer la composition sur les cils.
C'est pourquoi la présente invention a également pour objet un kit de maquillage et/ou de soin non thérapeutique des cils comprenant :

- un récipient contenant une composition sous forme de mousse présentant une densité inférieure ou égale à 0,95 et
- un applicateur comportant au moins un élément d'application configuré pour appliquer la composition sur les cils.

A titre d'applicateur, on peut citer notamment l'applicateur de type doigtier. En particulier un applicateur de type doigtier est prévu pour être monté à l'extrémité d'un doigt et comporte un état de surface présentant des reliefs formant saillie. Ces reliefs peuvent être disposés de manière régulière au niveau de cette surface. Ils peuvent être espacés d'une distance moyenne de l'ordre de 0,5 à 1 mm. Ces reliefs formant saillie peuvent avoir une hauteur de l'ordre de 1 mm relativement à la surface dont ils dépassent.
Pour prélever le produit sous forme de mousse, l'utilisatrice peut y plonger ces reliefs ponctuellement. Le produit est retenu entre ces reliefs et peut ensuite être appliqué sur une rangée de cils, les reliefs participant à l'étalement et au peignage de ces cils.
Selon un autre mode de réalisation avantageux, l'applicateur comprend un embout d'application.
En particulier, Figure 1, un mode de réalisation d'un applicateur 1 apte à être utilisé pour l'application d'une composition sous forme de mousse selon l'invention est représenté. Cet applicateur 1 comporte un embout applicateur 2 monté à l'extrémité d'un organe de préhension 3. Cet applicateur 1 peut être rangé, hors utilisation dans un logement 4 d'un organe de fermeture 5 d'un récipient 6 contenant le produit à appliquer. L'embout applicateur 2 peut être monté de manière amovible sur l'organe de préhension 3 de manière à pouvoir être changé. Le logement 4 peut comporter un essoreur. Il peut aussi comporter un siège imbibé destiné à être mis au contact de l'embout 2 pour éviter qu'il ne s'assèche ou pour permettre de le nettoyer, entre deux utilisations.
Dans cet exemple, le récipient 6 est un pot cylindrique tel que l'organe de fermeture 5 est monté par vissage sur un col de ce récipient. Par exemple, le vissage de l'organe de fermeture 5 est réalisé autour d'un axe Y, perpendiculaire à un plan dans lequel une ouverture du récipient 6 est définie. Le logement 4 configuré pour recevoir au moins l'embout applicateur 2 qui est dans cet exemple orienté selon un axe oblique Z relativement à l'axe Y. L'engagement de l'applicateur 1 dans son logement 4 est par exemple obtenu par translation. La rétention de l'applicateur dans son logement 4 est par exemple obtenue par encliquetage par coopération entre le moyen de préhension 3 et l'organe de fermeture 5.
De préférence, l'embout applicateur 2 comporte un axe longitudinal X, tel que l'insertion de l'applicateur 1 dans son logement 4 est réalisé par translation selon cet axe X, de telle sorte qu'en position rangée, l'axe Z du logement 4

et l'axe X de l'embout applicateur 2 soient sensiblement parallèles.

L'embout applicateur 2 est par exemple réalisé dans un matériau poreux. De préférence, la surface d'application définie par cet embout applicateur est configurée pour retenir un produit présenté sous forme de mousse, sans en dénaturer la structure, lorsque l'embout est amené au contact d'une surface de ce produit, ou au moins légèrement plongé dans ledit produit.

L'embout applicateur 2 peut être réalisé dans un matériau élastiquement déformable. Il peut être réalisé à partir d'un élastomère, d'un fritté, d'un feutre et ou d'une mousse. Le terme « feutre » signifie ici une structure fibreuse comportant des filaments enchevêtrés dans toutes les directions. La mousse est une structure alvéolaire qui peut comporter à sa surface des cratères permettent d'emmagasiner et retenir en surface une quantité de produit, ces cratères pouvant être répartis de manière régulière ou irrégulière en surface. La mousse peut être à cellules ouvertes, semi ouvertes ou fermées. De préférence, le diamètre des cratères à la surface de l'embout applicateur 2 est sensiblement égal ou supérieur au diamètre moyen des bulles d'air formées dans le produit sous forme de mousse.

La mousse de l'embout applicateur est par exemple réalisée à partir de l'un au moins des matériaux choisis dans la liste suivante : polyuréthane, polyéther, polyester, polychlorure de vinyle, EVA, cette liste n'étant pas limitative.

L'embout applicateur 2 peut être recouvert d'un flocage, par exemple déposé sur une couche d'un matériau thermoplastique. Le flocage peut être, par exemple, constitué de poils de différents diamètres, et/ou nature, et/ou hauteur ou d'un mélange de tels poils. L'embout applicateur 2 peut être recouvert d'un tissé ou d'un non-tissé ou de tout autre revêtement présentant des reliefs vers l'extérieur, par exemple des crochets ou des boucles formant saillie vers l'extérieur du revêtement.

L'embout applicateur 2 peut être une structure multicouche comportant une couche définissant une surface d'application et une ou plusieurs couches inférieures destinées par exemple à conférer une rigidité ou une souplesse plus ou moins grande à l'embout applicateur et/ ou à améliorer la capacité d'absorption de produit par l'applicateur.

L'embout applicateur 2 est par exemple de forme tubulaire de section transversale circulaire. En variantes représentées Figures 2 à 5, et sans sortir du cadre de la présente invention, la section transversale de l'embout applicateur 2 peut aussi être ovale ou polygonale, notamment triangulaire, carrée ou rectangulaire.

Une extrémité libre 7 de cet embout applicateur 2, opposée à l'extrémité de l'embout retenue dans l'organe de préhension 3, peut être sensiblement définie dans un plan. Cette extrémité libre 7 peut définir au moins en partie une surface destinée à être chargée en produit et à être mise au contact des cils.

Par exemple, cette extrémité libre 7 est définie dans un plan perpendiculaire à l'axe longitudinal X, Figures 2 et 4, ou bien être définie dans un plan oblique, différent de perpendiculaire, avec cet axe X, comme représenté aux Figures 1 et 5. En variante encore, cette extrémité libre 7 peut ne pas être plane et peut présenter au moins une concavité ou une convexité. Notamment, Figure 3, l'extrémité libre 7 forme globalement un dôme, elle est alors convexe vers l'extérieur.

L'intérêt d'une portion concave vers l'extérieur est qu'elle permet d'y retenir une quantité de produit, notamment lorsqu'une telle concavité est trempée dans le produit ici sous forme de mousse. A cet effet, dans les modes de réalisation des Figures 2 à 5, l'embout applicateur 2 comporte une encoche 8 (aussi appelée échancrure), concave vers l'extérieur, définie au travers de son extrémité libre 7. L'embout applicateur 2 comporte de préférence au moins une encoche telle que 8.

De préférence, l'applicateur comprend un embout d'application dont une extrémité libre présente au moins une concavité, sous forme notamment d'une échancrure.

Un autre intérêt de cette encoche 8, tient au fait qu'elle est configurée pour recevoir une portion d'une frange de cils, et qu'elle permet d'enduire et d'agencer les cils de cette portion de telle sorte qu'ils se rejoignent au niveau de leurs extrémités libres. En effet, des cils disposés dans cette encoche 8, comme représenté Figure 6, sont enduits de telle sorte que le déplacement en translation de l'extrémité libre 7 à leur contact les force à s'agglomérer en crête. Le maquillage se fait en commençant par la base des cils et en effectuant un mouvement en translation simultané vers la pointe des cils et vers le fond de l'encoche 8.

Selon une vue en coupe perpendiculairement à l'extrémité libre 7, cette encoche 8 est de section triangulaire, de telle sorte que la base de ce triangle soit choisie en fonction de la largeur de la portion de cils que l'on souhaite maquiller et arranger sous forme de crête. La hauteur de ce triangle est configurée pour permettre un enfoncement des cils dans cette encoche 8 sur au moins 50% de cette hauteur de manière à obtenir lesdites crêtes.

Selon les modes de réalisation représentés, l'encoche 8 présente une section triangulaire selon un plan de coupe P parallèle à l'axe X choisi de manière à couper les deux bords latéraux 9 et 10 de ladite encoche 8. En particulier, le fond de l'encoche 8 forme une arête 11 qui est linéaire. Figures 2 à 5, cette arête 11 est une droite. En variante, cette arête 11 pourrait être courbe de manière à faire varier la profondeur de l'encoche 8 relativement à la surface d'application définie par l'extrémité libre 7.

Le contenu des brevets ou demandes de brevets cités précédemment est incorporés par référence dans la présente demande.

Les exemples qui suivent sont présentés à titre illustratif et non limitatif de l'invention. Sauf indication contraire,

les quantités sont données en pourcentage en poids.

**Exemples 1 à 3**

On prépare les mascaras suivants selon l'invention :

| | **Exemple 1 (%)** | **Exemple 2 (%)** | **Exemple 3 (%)** |
|---|---|---|---|
| Cire d'abeille | 22,5 | 32,2 | 28,5 |
| Sucrostéarate | 4,5 | 5,35 | 4,75 |
| Monostéarate de PEG-40 (MYRJ 52P® de ICI UNIQUEMA) | 1,75 | 2,08 | 1,85 |
| Tristéarate de sorbitan (SPAN 65V d'UNIQUEMA) | 0,77 | 0,9 | 0,8 |
| Huile d'amande d'abricot | 4,5 | - | - |
| Noir de carbone | 2,7 | - | - |
| Oxyde de fer noir | - | 8 | 7,1 |
| Sodium laureth sulfate | 4,5 | 4,8 | 4,75 |
| Copolymère acide polyacryliques/acrylates d'alkyle en C10-C30 réticulé ( PEMULEN TR-2 de NOVEON) | 0,27 | 0,29 | - |
| Gomme de xanthane | - | 0,11 | - |
| Farine de caroube | - | 0,15 | - |
| Alginate | - | - | 0,49 |
| CaCl2 | - | - | 0,1 |
| conservateurs | Qs | Qs | Qs |
| eau | Qsp100 | Qsp100 | Qsp100 |

*Mode opératoire*

On prépare l'émulsion à l'agitateur Moritz : on chauffe les composés de la phase grasse à une température d'environ 90°C. La phase aqueuse est ensuite versée sur la phase grasse sous agitation Moritz. L'agitation est maintenue 10 minutes à chaud.

L'émulsion est ensuite versée dans un batteur Kenwood. Lorsque la température de l'émulsion atteint environ 60°C lors du refroidissement, on la foisonne pendant 5 minutes

Pour chacune des compositions, on a mesuré les paramètres de densité, de module de rigidité et d'extrait sec, selon les protocoles décrits plus haut.

Les résultats sont présentés dans les tableaux suivants :

| | Densité | Gp (Pa) | Extrait sec (%) |
|---|---|---|---|
| **Exemple 1** | 0,35 | 350 | 41,6 |
| **Exemple 2** | 0,5 | 1600 | 55,4 |
| **Exemple 3** | 0,66 | 23600 | 49,5 |

**Exemple 4**

On prépare le mascara suivant selon l'invention :

| | Exemple 4 |
|---|---|
| Cire de carnauba | 25 |
| Sucrostéarate | 5 |

(suite)

| | Exemple 4 |
|---|---|
| Monostéarate de PEG-40 (MYRJ 52P® de ICI UNIQUEMA) | 1,95 |
| Tristéarate de sorbitan (SPAN 65V d'UNIQUEMA) | 0,85 |
| Huile d'amande d'abricot | 5 |
| Oxyde de fer noir | 3 |
| Sodium laureth sulfate | 5 |
| Copolymère acide polyacryliques/acrylates d'alkyle en C10-C30 réticulé ( PEMULEN TR-2 de NOVEON) | 0,3 |
| Conservateurs | qs |
| Eau | Qsp 100% |

Mode opératoire :

On chauffe les composés de la phase grasse à une température d'environ 90˚C. La phase aqueuse est ensuite versée sur la phase grasse sous agitation Moritz. L'agitation est maintenue 10 minutes à chaud.

L'émulsion est ensuite versée dans un batteur Kenwood. Lorsque la température de l'émulsion atteint environ 80˚C lors du refroidissement, on la foisonne pendant 2 minutes

On a mesuré les paramètres de densité, de module de rigidité et d'extrait sec de la composition, selon les protocoles décrits plus haut.

| Densité | Gp (Pa) | Extrait sec (%) |
|---|---|---|
| 0,73 | 25000 | 45,9 |

**Revendications**

1. Procédé de revêtement des cils comprenant l'application sur lesdits cils d'au moins une couche d'au moins une composition sous forme de mousse, ladite composition présentant une densité inférieure ou égale à 0,95 et un module de rigidité plateau Gp inférieur à 50 000 Pa.

2. Procédé de revêtement des cils comprenant l'application sur lesdits cils d'au moins une couche d'au moins une composition sous forme de mousse, ladite composition présentant une densité inférieure ou égale à 0,95 et une teneur en matière sèche inférieure ou égale à 60 % en poids par rapport au poids total de la composition.

3. Procédé de revêtement des cils comprenant l'application sur lesdits cils d'au moins une couche d'au moins une composition sous forme de mousse, ladite composition présentant une densité inférieure ou égale à 0,95 et comprenant une phase grasse liquide et au moins un agent structurant de phase grasse liquide choisi parmi les corps gras pâteux, les polymères semi-cristallins, les gélifiants lipophiles, et leurs mélanges.

4. Procédé de revêtement cils comprenant l'application sur lesdits cils d'au moins une couche d'au moins une composition sous forme de mousse, ladite composition présentant une densité inférieure ou égale à 0,95 et comprenant une phase aqueuse et au moins un gélifiant hydrophile.

5. Procédé selon l'une des revendications précédentes, **caractérisé en ce que** la composition présente une densité inférieure ou égale à 0,9.

6. Procédé selon l'une des revendications 1, 2 ou 4, **caractérisé en ce que** la composition comprend une phase grasse liquide.

7. Procédé selon la revendication précédente 3 ou 6, **caractérisé en ce que** la phase grasse liquide est présente en une teneur allant de 0,1% à 30 % en poids, de préférence de 1 % à 20 % en poids par rapport au poids total de la

composition.

**8.** Procédé selon la revendication 6, **caractérisé en ce que** la composition comprend au moins un agent structurant de la phase grasse liquide choisi parmi les corps gras pâteux, les polymères semi-cristallins, les gélifiants lipophiles et leurs mélanges.

**9.** Procédé selon la revendication 3 ou 8, **caractérisé en ce que** l'agent structurant est présent en une teneur variant de 0,1 à 60 % en poids par rapport au poids total de la composition, de préférence de 0,5 à 50 % et de façon encore plus préférée de 1 à 40 % en poids.

**10.** Procédé selon l'une quelconque des revendications 1 à 3, **caractérisé en ce que** la composition comprend une phase aqueuse.

**11.** Procédé selon la revendication 4 ou 10, **caractérisé en ce** la phase aqueuse est présente, en une teneur allant de 1 % à 95 % en poids, par rapport au poids total de la composition, de préférence allant de 3 % à 80 % en poids, et préférentiellement allant de 5 % à 60 % en poids.

**12.** Procédé selon l'une quelconque des revendications 1 à 3 et 5 à 11, **caractérisé en ce que** la composition comprend au moins un gélifiant hydrophile.

**13.** Procédé selon la revendication 4 ou 12, **caractérisé en ce que** le gélifiant hydrophile est choisi parmi
les homo- ou copolymères d'acides acrylique ou méthacrylique ou leurs sels et leurs esters,
les copolymères d'acide acrylique et d'acrylamide sous forme de leur sel de sodium, les sels de sodium d'acides polyhydroxycarboxyliques,
les copolymères acide polyacryliques/acrylates d'alkyle de type,
l'AMPS (Acide polyacrylamidométhyl propane sulfonique neutralisé partiellement à l'ammoniaque et hautement réticulé),
les copolymères AMPS/acrylamide, et
les copolymères AMPS/méthacrylates d'alkyle polyoxyéthylénés (réticulés ou non),
les protéines d'origine végétale ou animale ;
les polymères de cellulose ainsi que les dérivés quaternisés de la cellulose ;
les polymères ou copolymères acryliques, tels que les polyacrylates ou les polyméthacrylates ;
les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
les polymères d'origine naturelle, éventuellement modifiés, tels que :

les gommes arabiques, la gomme de guar, les dérivés du xanthane, la gomme de karaya, la gomme de caroube ;
les alginates et les carraghénanes ;
les glycoaminoglycanes, l'acide hyaluronique et ses dérivés ;
la résine shellac, la gomme de sandaraque, les dammars, les élémis, les copals ;
l'acide désoxyribonucléïque ;
les muccopolysaccharides tels les chondroïtines sulfate,
et leurs mélanges.

**14.** Procédé selon la revendication 4 ou 12, **caractérisé en ce que** le gélifiant hydrophile est présent en une teneur en matières sèches allant de 0,01% à 30%, mieux 0,05% à 20%, voire 0,1 % à 10% en poids par rapport au poids total de la composition.

**15.** Procédé selon la revendication 4 ou 10, **caractérisé en ce que** la composition comprend un système émulsionnant.

**16.** Procédé selon la revendication 4 ou 15, **caractérisé en ce que** le système émulsionnant est présent en une proportion allant de 0,1 à 20 %, et mieux de 0,3 % à 15 % en poids par rapport au poids total de la composition.

**17.** Procédé selon l'une des revendications précédentes, **caractérisé en ce que** la composition comprend au moins une cire.

**18.** Procédé selon la revendication 17, **caractérisé en ce que** la cire est présente en une teneur allant de 0,1 à 50 %

en poids par rapport au poids total de la composition, en particulier de 0,5 à 35 % en poids.

**19.** Procédé selon l'une des revendications précédentes, **caractérisé en ce que** la composition comprend une matière colorante.

**20.** Procédé selon la revendication 19, **caractérisée en ce que** la matière colorante représente de 0,01 à 30 % en poids par rapport au poids total de la composition.

**21.** Kit de maquillage et/ou de soin non thérapeutique des cils comprenant :

- un récipient contenant une composition sous forme de mousse présentant une densité inférieure ou égale à 0,95 et
- un applicateur comportant au moins un élément d'application configuré pour appliquer la composition sur les cils.

**22.** Kit selon la revendication 21, **caractérisé en ce que** l'applicateur est de type doigtier.

**23.** Kit selon la revendication 21, **caractérisé en ce que** l'applicateur comprend un embout d'application.

**24.** Kit selon la revendication 23, **caractérisé en ce que** une extrémité libre de l'embout d'application présente au moins une concavité, sous forme notamment d'une échancrure.

## Claims

**1.** Process for coating the eyelashes, comprising the application, to the said eyelashes, of at least one layer of at least one composition in the foam form, the said composition exhibiting a density of less than or equal to 0.95 and a plateau rigidity modulus Gp of less than 50 000 Pa.

**2.** Process for coating the eyelashes, comprising the application, to the said eyelashes, of at least one layer of at least one composition in the foam form, the said composition exhibiting a density of less than or equal to 0.95 and a content of dry matter of less than or equal to 60% by weight, with respect to the total weight of the composition.

**3.** Process for coating the eyelashes, comprising the application, to the said eyelashes, of at least one layer of at least one composition in the foam form, the said composition exhibiting a density of less than or equal to 0.95 and comprising a liquid fatty phase and at least one liquid-fatty-phase structuring agent chosen from pasty fatty substances, semicrystalline polymers, lipophilic gelling agents and their mixtures.

**4.** Process for coating the eyelashes, comprising the application, to the said eyelashes, of at least one layer of at least one composition in the foam form, the said composition exhibiting a density of less than or equal to 0.95 and comprising an aqueous phase and at least one hydrophilic gelling agent.

**5.** Process according to one of the preceding claims, **characterized in that** the composition exhibits a density of less than or equal to 0.9.

**6.** Process according to one of Claims 1, 2 or 4, **characterized in that** the composition comprises a liquid fatty phase.

**7.** Process according to Claim 3 or 6, **characterized in that** the liquid fatty phase is present in a content ranging from 0.1% to 30% by weight, preferably from 1% to 20% by weight, with respect to the total weight of the composition.

**8.** Process according to Claim 6, **characterized in that** the composition comprises at least one liquid-fatty-phase structuring agent chosen from pasty fatty substances, semicrystalline polymers, lipophilic gelling agents and their mixtures.

**9.** Process according to Claim 3 or 8, **characterized in that** the structuring agent is present in a content varying from 0.1 to 60% by weight, with respect to the total weight of the composition, preferably from 0.5 to 50% by weight and more preferably still from 1 to 40% by weight.

10. Process according to any one of Claims 1 to 3, **characterized in that** the composition comprises an aqueous phase.

11. Process according to Claim 4 or 10, **characterized in that** the aqueous phase is present in a content ranging from 1% to 95% by weight, with respect to the total weight of the composition, preferably ranging from 3% to 80% by weight and preferentially ranging from 5% to 60% by weight.

12. Process according to any one of Claims 1 to 3 and 5 to 11, **characterized in that** the composition comprises at least one hydrophilic gelling agent.

13. Process according to Claim 4 or 12, **characterized in that** the hydrophilic gelling agent is chosen from
homo- or copolymers of acrylic acid or methacrylic acid or their salts and their esters,
copolymers of acrylic acid and of acrylamide in the form of their sodium salt,
sodium salts of poly(hydroxycarboxylic acid)s,
poly(acrylic acid)/alkyl acrylate copolymers,
AMPS (poly(acrylamidomethylpropanesulphonic acid) partially neutralized with aqueous ammonia and highly crosslinked),
AMPS/acrylamide copolymers, and
copolymers of AMPS and of alkyl methacrylates which are polyoxyethylenated (crosslinked or noncrosslinked),
proteins of plant or animal origin;
cellulose polymers and quaternized cellulose derivatives;
acrylic polymers or copolymers, such as polyacrylates or polymethacrylates;
vinyl polymers, such as polyvinylpyrrolidones, copolymers of methyl vinyl ether and of malic anhydride, the copolymer of vinyl acetate and of crotonic acid, copolymers of vinylpyrrolidone and of vinyl acetate, copolymers of vinylpyrrolidone and of caprolactam, or poly(vinyl alcohol);
optionally modified polymers of natural origin, such as:

gums arabic, guar gum, xanthan derivatives, karaya gum or locust bean gum;
alginates and carrageenans;
glycoaminoglycans, hyaluronic acid and its derivatives;
shellac resin, gum sandarac, dammars, elemis or copals;
deoxyribonucleic acid;
mucopolysaccharides, such as chondroitin sulphates,
and their mixtures.

14. Process according to Claim 4 or 12, **characterized in that** the hydrophilic gelling agent is present in a content of dry matter ranging from 0.01% to 30%, better still 0.05% to 20%, indeed even 0.1% to 10%, by weight, with respect to the total weight of the composition.

15. Process according to Claim 4 or 10, **characterized in that** the composition comprises an emulsifying system.

16. Process according to Claim 4 or 15, **characterized in that** the emulsifying system is present in a proportion ranging from 0.1% to 20% and better still from 0.3% to 15% by weight, with respect to the total weight of the composition.

17. Process according to one of the preceding claims, **characterized in that** the composition comprises at least one wax.

18. Process according to Claim 17, **characterized in that** the wax is present in a content ranging from 0.1 to 50% by weight, with respect to the total weight of the composition, in particular from 0.5 to 35% by weight.

19. Process according to one of the preceding claims, **characterized in that** the composition comprises a colouring material.

20. Process according to Claim 19, **characterized in that** the colouring material represents from 0.01 to 30% by weight, with respect to the total weight of the composition.

21. Kit for making up and/or for the nontherapeutic care of the eyelashes comprising:

- a container comprising a composition in the foam form exhibiting a density of less than or equal to 0.95 and
- an applicator comprising at least one application component configured to apply the composition to the eyelashes.

**22.** Kit according to Claim 21, **characterized in that** the applicator is of fingerstall type.

**23.** Kit according to Claim 21, **characterized in that** the applicator comprises an application nozzle.

**24.** Kit according to Claim 23, **characterized in that** a free end of the application nozzle exhibits at least one concavity in the form in particular of an indentation.

**Patentansprüche**

**1.** Verfahren zum Beschichten von Wimpern, das das Aufbringen zumindest einer Schicht mindestens einer Zusammensetzung in Form von Schaum auf die Wimpern umfasst, wobei die Zusammensetzung eine Dichte kleiner oder gleich 0,95 und einen Plateau-Steifigkeitsmodul Gp unter 50 000 Pa besitzt.

**2.** Verfahren zum Beschichten von Wimpern, das das Aufbringen zumindest einer Schicht mindestens einer Zusammensetzung in Form von Schaum auf die Wimpern umfasst, wobei die Zusammensetzung eine Dichte kleiner oder gleich 0,95 aufweist und einen Trockensubstanzgehalt kleiner oder gleich 60 Gew.-%, bezogen auf das Gesamtgewicht der Zusammensetzung, besitzt.

**3.** Verfahren zum Beschichten von Wimpern, das das Aufbringen zumindest einer Schicht mindestens einer Zusammensetzung in Form von Schaum auf die Wimpern umfasst, wobei die Zusammensetzung eine Dichte kleiner oder gleich 0,95 aufweist und mindestens eine flüssige Fettphase und mindestens ein Strukturierungsmittel für die flüssige Fettphase enthält, das unter den pastösen Fettsubstanzen, halbkristallinen Polymeren, lipophilen Gelbildnern und deren Gemischen ausgewählt ist.

**4.** Verfahren zum Beschichten von Wimpern, das das Aufbringen zumindest einer Schicht mindestens einer Zusammensetzung in Form von Schaum auf die Wimpern umfasst, wobei die Zusammensetzung eine Dichte kleiner oder gleich 0,95 aufweist und eine wässrige Phase und mindestens einen hydrophilen Gelbildner enthält.

**5.** Verfahren nach einem der vorhergehenden Ansprüche, **dadurch gekennzeichnet, dass** die Zusammensetzung eine Dichte kleiner oder gleich 0,9 aufweist.

**6.** Verfahren nach einem der Ansprüche 1, 2 oder 4, **dadurch gekennzeichnet, dass** die Zusammensetzung eine flüssige Fettphase enthält,

**7.** Verfahren nach dem vorhergehenden Anspruch 3 oder 6, **dadurch gekennzeichnet, dass** die flüssige Fettphase in einem Mengenanteil von 0,1 bis 30 Gew.-% und vorzugsweise 1 bis 20 Gew.-%, bezogen auf das Gesamtgewicht der Zusammensetzung, vorliegt.

**8.** Verfahren nach Anspruch 6, **dadurch gekennzeichnet, dass** die Zusammensetzung mindestens ein Strukturierungsmittel für die flüssige Fettphase enthält, das unter den pastösen Fettsubstanzen, halbkristallinen Polymeren, lipophilen Gelbildnern und deren Gemischen ausgewählt ist.

**9.** Verfahren nach Anspruch 3 oder 8, **dadurch gekennzeichnet dass** das Strukturierungsmittel in einem Mengenanteil von 0,1 bis 60 Gew.-%, bezogen auf das Gesamtgewicht der Zusammensetzung, vorzugsweise 0,5 bis 50 Gew.-% und noch bevorzugter 1 bis 40 Gew,-% enthalten ist.

**10.** Verfahren nach einem der Ansprüche 1 bis 3, **dadurch gekennzeichnet, dass** die Zusammensetzung eine wässrige Phase enthält.

**11.** Verfahren nach Anspruch 4 oder 10, **dadurch gekennzeichnet, dass** die wässrige Phase in einem Mengenanteil von 1 bis 95 Gew.-%, bezogen auf das Gesamtgewicht der Zusammensetzung, vorzugsweise 3 bis 80 Gew.-% und noch bevorzugter 5 bis 60 Gew.-% enthalten ist.

**12.** Verfahren nach einem der Ansprüche 1 bis 3 und 5 bis 11, **dadurch gekennzeichnet, dass** die Zusammensetzung mindestens einen hydrophilen Gelbildner enthält.

**13.** Verfahren nach Anspruch 4 oder 12, **dadurch gekennzeichnet, dass** der hydrophile Gelbildner ausgewählt ist unter
Homo- oder Copolymeren von Acrylsäure oder Methacrylsäure oder deren Salzen und deren Estern,
Copolymeren von Acrylsäure und Acrylamid in Form ihres Natriumsalzes,
Natriumsalzen von Polyhydroxycarbonsäuren,
Polyacrylsäure/Alkylacrylate-Copolymeren,
AMPS (Polyacrylamidomethylpropansulfonsäure, zum Teil mit Ammoniak neutralisiert und hochvernetzt),
AMPS/Acrylamid-Copolymeren und
AMPS/polyethoxylierte (vernetze oder nichtvernetzte) Alkylmethacrylate-Copolymeren,
Proteinen pflanzliche oder tierischer Herkunft;
Polymeren von Cellulose sowie quaternisierten Cellulosederivaten;
Acrylpolymeren oder Acrylcopolymeren, wie Polyacrylaten oder Polymethacrylaten;
Vinylpolymeren, wie Polyvinylpyrrolidonen, Copolymeren von Methylvinylether und Maleinsäureanhydrid, dem Copolymer von Vinylacetat und Crotonsäure, Copolymeren von Vinylpyrrolidon und Vinylacetat; Copolymeren von Vinylpyrrolidon und Caprolactam; Polyvinylalkohol;
Polymeren natürlicher Herkunft, die gegebenenfalls modifiziert sind, wie beispielsweise:

Gummi arabicum, Guargummi, Xanthanderivaten, Karaya-Gummi, Johannisbrotkernmehl;
Alginaten und Carrageenanen;
Glycosaminoglycanen, Hyaluronsäure und ihren Derivaten;
Schellack, Sandarak, Dammarharzen, Elemi, Kopalen; Desoxyribonucleinsäure;
Mucopolysacchariden, wie Chondroitinsulfaten;
und deren Gemischen,

**14.** Verfahren nach Anspruch 4 oder 12, **dadurch gekennzeichnet, dass** der hydrophile Gelbildner in einem Trockensubstanzgehalt von 0,01 bis 30 %, besser 0,05 bis 20 % und sogar 0,1 bis 10 Gew.-%, bezogen auf das Gesamtgewicht der Zusammensetzung, vorliegt,

**15.** Verfahren nach Anspruch 4 oder 10, **dadurch gekennzeichnet, dass** die Zusammensetzung ein Emulgatorsystem enthält.

**16.** Verfahren nach Anspruch 4 oder 15, **dadurch gekennzeichnet, dass** das Emulgatorsystem in einem Mengenanteil von 0,1 bis 20 % und besser 0,3 bis 15 Gew.-%, bezogen auf das Gesamtgewicht der Zusammensetzung, enthalten ist.

**17.** Verfahren nach einem der vorhergehenden Ansprüche, **dadurch gekennzeichnet, dass** die Zusammensetzung mindestens ein Wachs enthält.

**18.** Verfahren nach Anspruch 17, **dadurch gekennzeichnet, dass** das Wachs in einem Mengenanteil von 0,1 bis 50 Gew.-%, bezogen auf das Gesamtgewicht der Zusammensetzung, und insbesondere 0,5 bis 35 Gew.-% enthalten ist.

**19.** Verfahren nach einem der vorhergehenden Ansprüche, **dadurch gekennzeichnet, dass** die Zusammensetzung ein Farbmittel enthält.

**20.** Verfahren nach Anspruch 19, **dadurch gekennzeichnet, dass** das Farbmittel 0,01 bis 30 Gew.-%, bezogen auf das Gesamtgewicht der Zusammensetzung, ausmacht.

**21.** Kit zum Schminken und/ oder für die nichttherapeutische Pflege der Wimpern, umfassend:

- einen Behälter, der einer Zusammensetzung in Form von Schaum mit einer Dichte kleiner oder gleich 0,95 enthält und
- einen Applikator, der mindestens ein Applikationselement umfasst, das für das Aufbringen der Zusammensetzung auf die Wimpern ausgelegt ist.

**22.** Kit nach Anspruch 21, **dadurch gekennzeichnet, dass** der Applikator vom Typ eines Fingerlings ist.

**23.** Kit nach Anspruch 21, **dadurch gekennzeichnet, dass** der Applikator eine Auftrageeinheit umfasst.

**24.** Kit nach Anspruch 23, **dadurch gekennzeichnet, dass** das freie Ende der Auftrageeinheit mindestens eine Vertiefung insbesondere in der Form eines Einschnitts besitzt.

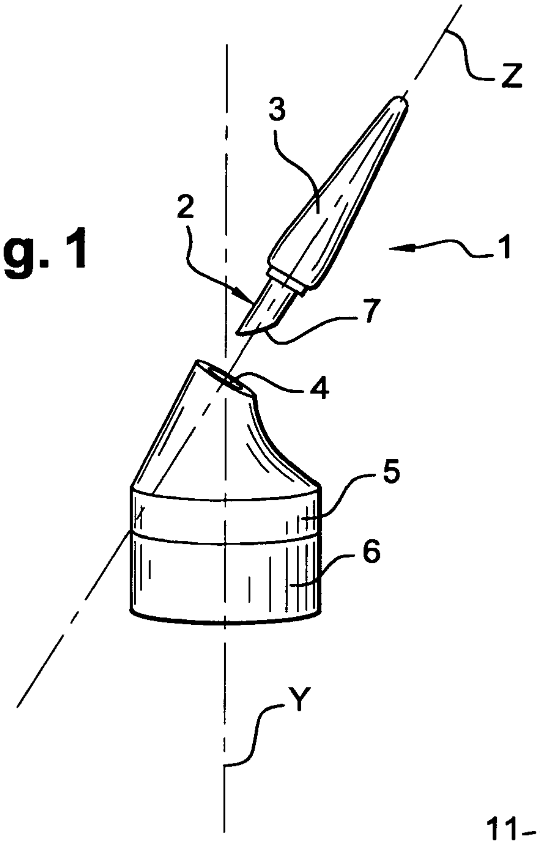


Fig. 1

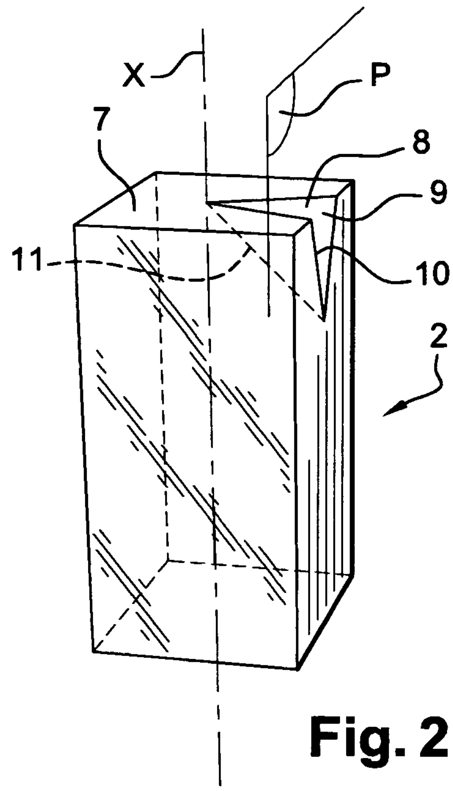


Fig. 2

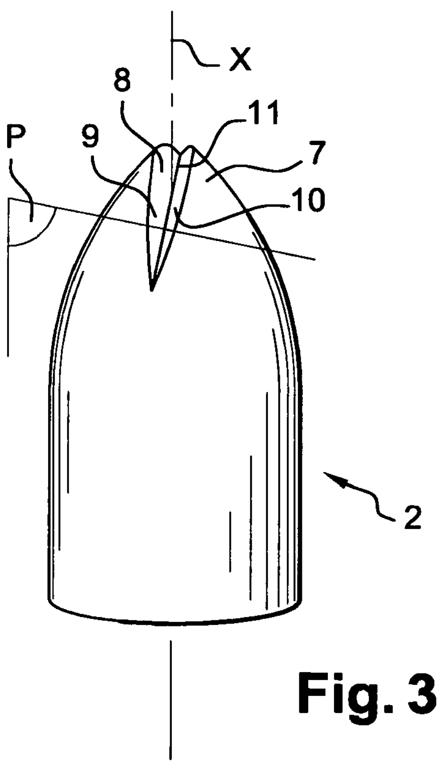


Fig. 3

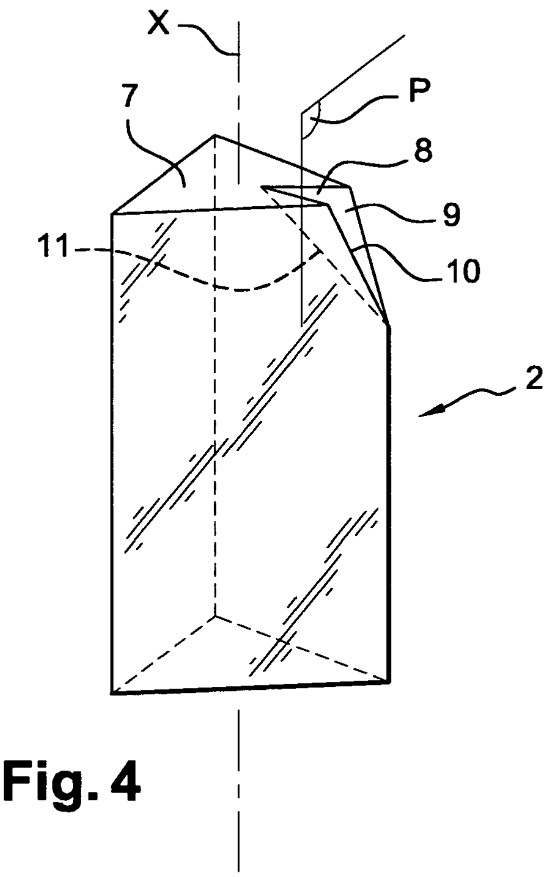


Fig. 4

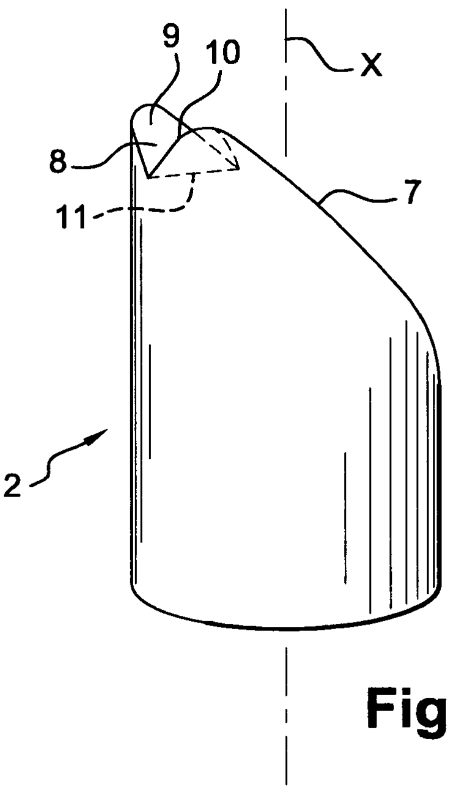


Fig. 5

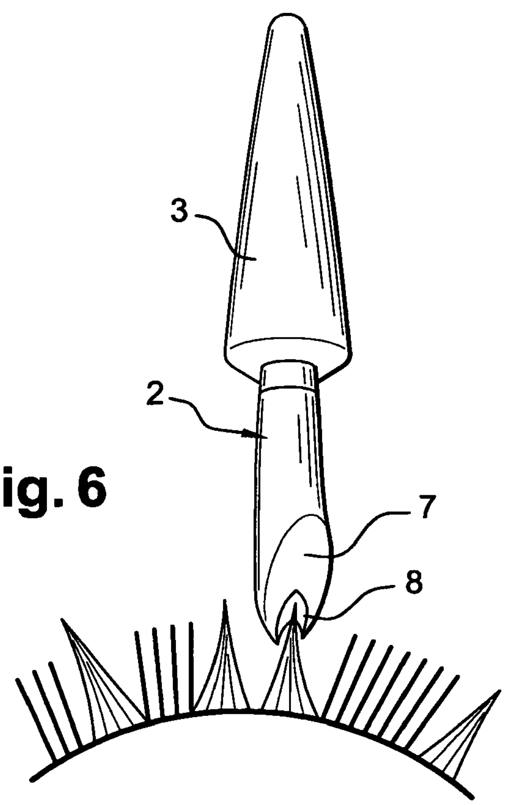


Fig. 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1407755 A
- US 2007245 A
- FR 2833163
- WO 2004060292 A2
- EP 847752 A
- EP 1396259 A
- US 5874069 A
- US 5919441 A
- US 6051216 A
- US 5981680 A
- FR 2792190 A
- FR 2232303 A
- US 5162410 A
- WO 2004073626 A
- EP 1411069 A
- WO 04028488 A
- WO 04055081 A


### Littérature non-brevet citée dans la description


- **G. COUARRAZE ; J.L. GROSSIORD.** Initiation à la rhéologie. 1991
- Silica diméthyl silylate. 1995
- *J. Soc. Cosm. Chem.,* 1954, vol. 5, 249-256
- **KIRK-OTHMER.** Encyclopedia of Chemical Technology. WILEY, 1979, vol. 22, 333-432
- **VAN DE HULST, H.C.** Light Scattering by Small Particles. Wiley, 1957